# EUROPEAN PATENT SPECIFICATION

(11) **EP 1 031 567 B1**
(45) Date of publication and mention of the grant of the patent: **02.04.2003**
(21) Application number: 98950461.8
(22) Date of filing: 30.10.1998
(51) Int. Cl.: C07D 285/06, A01N 43/82

(54) **THIADIAZOLE CARBOXAMIDE DERIVATIVE, PLANT DISEASE CONTROLLING AGENT, AND METHOD OF USING THE SAME**
THIADIAZOLCARBOXAMID-DERIVAT, MITTEL ZUR BEKÄMPFUNG VON PFLANZENKRANKHEITEN UND VERFAHREN ZUR ANWENDUNG
DERIVE DE THIADIAZOLE CARBOXAMIDE, AGENT PHYTOSANITAIRE, ET UTILISATION

(30) Priority: 31.10.1997 JP 31642797
(43) Date of publication of application: 30.08.2000
(73) Proprietor: Nihon Nohyaku Co., Ltd., Tokyo 103-0027 (JP)
(72) Inventor: TSUBATA, Kenji, Kawachinagano-shi Osaka 586-0076 (JP); SANPEI, Osamu, Kawachinagano-shi Osaka 586-0022 (JP); TAKAGI, Kazuhiro, Osaka-shi Osaka 550-0014 (JP); UMETANI, Kunihisa, Osakasayama-shi Osaka 589-0011 (JP); UCHIKUROHANE, Tohru, Hashimoto-shi Wakayama 648-0018 (JP); TAJIMA, Sohkichi, Osaka-shi Osaka 558-0003 (JP)
(74) Representative: Grünecker, Kinkeldey, Stockmair & Schwanhäusser Anwaltssozietät
(86) International application number: JP9804918
(87) International publication number: WO99023084

(56) References cited:
- JP-A- 8 325 110
- JP-A- 54 009 272
- JP-A- 57 067 567

## Description

The present invention relates to thiadiazole carboxamide derivatives, a plant disease controller and its usage.

### BACKGROUND ART

JP-A-8-325110 describes 1,2,3-thiadiazole derivatives including the compounds of the present invention as useful as a plant disease controller. This reference, however, does not disclose N-(3-chloro-4-methylphenyl)-4-methyl-1,2,3-thiadiazole-5-carboxamide of the present invention at all.

### DISCLOSURE OF THE INVENTION

The present inventors earnestly investigated in order to develop a novel plant disease controller, and consequently found that N-(3-chloro-4-methylphenyl)-4-methyl-1,2,3-thiadiazole-5-carboxamide of the present invention is a novel compound not known in any literature and that it is a plant disease controller which is more effective in a low dosage against, in particular, diseases of rice (in particular, blast), barley, wheat, oats, rye, vegetables, etc., has less phytotoxicity and is more harmless to mammals than the compounds disclosed in JP-A-8-325110, whereby the present invention has been accomplished. That is, the present invention relates to N-(3-chloro-4-methylphenyl)-4-methyl-1,2,3-thiadiazole-5-carboxamide, a plant disease controller containing this compound as an active ingredient, and a usage of the plant disease controller.

N-(3-chloro-4-methylphenyl)-4-methyl-1,2,3-thiadiazole-5-carboxamide of the present invention can be produced, for example, by the production process disclosed in JP-A-8-325110.

N-(3-chloro-4-methylphenyl)-4-methyl-1,2,3-thiadiazole-5-carboxamide of the present invention is useful as a plant desease controller and is very effective in controlling various diseases, for example, bacterial diseases, e.g., rice blast (Pyricularia oryzae), rice sheath blight (Rhizoctonia solani), rice helminthosporium leaf spot (Cochiobolus miyabeanus), powdery mildew of various host plants, such as powdery mildew of barley and wheat (Erysiphe graminis), oats crown rust (Puccinia coronata), rust of other plants, tomato late blight (Phytophthora infestans), late blight or Phytophthora rots of other plants, downy mildew of various plants, such as cucumber downy mildew (Pseudoperonospora cubensis) and grape downy mildew (Plasmopara viticola), apple scab (Venturia inaequalis), apple alternaria leaf spot (Alternaria mali), pear black spot (Alternaria kikuchiana), citrus melanose (Diaporthe citri), diseases caused by Pseudomonas bacteria, such as cucumber bacterial spot (Pseudomonas syringae pv. lachrymans) and tomato bacterial wilt (Pseudomonas solanacearum), diseases caused by Xanthomonas bacteria, such as cabbage black rot (Xanthomonas campestris), rice bacterial leaf blight (Xanthomonas oryzae), citrus bacterial canker (Xanthomonas citri), and diseases caused by Erwinia bacteria, such as cabbage bacterial soft rot (Erwinia carotovora); and viral diseases, for example, tobacco mosaic (Tobacco mosaic virus). Said compound is a plant disease controller having a high controlling activity against, in particular, rice blast and no phytotoxicity to rice.

The agricultural and horticultural disease controller containing N-(3-chloro-4-methylphenyl)-4-methyl-1,2,3-thiadiazole-5-carboxamide of the present invention as an active ingredient is markedly effective in controlling the above-exemplified diseases which damage paddy field crops, upland crops, fruit trees, vegetables, other crops, flowers and ornamental plants, and the like. Therefore, the desired effects of the agricultural and horticultural disease controller of the present invention can be obtained by applying the disease controller to the paddy field water, stalks and leaves of fruit trees, vegetables, other crops, flowers and ornamental plants, soil, etc., at a season at which the diseases are expected to occur, before their occurrence or at the time when their occurrence is confirmed.

In general, the agricultural and horticultural disease controller of the present invention is used after being prepared into a conveniently usable form according to an ordinary manner for preparation of agrochemicals.

That is, N-(3-chloro-4-methylphenyl)-4-methyl-1,2,3-thiadiazole-5-carboxamide of the present invention and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in the present invention may be either solid or liquid. As the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain calcium silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphtha and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The plant disease controller of the present invention is used to control various diseases in the following manner. That is, it is applied to a crop on which the diseases are expected to occur, or a site where the occurrence of the diseases is undesirable, as it is or after being properly diluted with or suspended in water or the like, in an amount effective for control of the diseases. For example, to control the diseases of paddy rice, said disease controller can be used by a method such as submerged application to a regular paddy field, application to a rice nursery box at any of various application times such as the day of transplantation, greening stage, and a period between bed soil mixing and soil covering, dressing of seeds for direct sowing on flooded paddy field, or seed disinfection. To control diseases of barley, wheat, oats, rye, vegetables and the like, said plant disease controller can be used not only by spraying on the stalks and leaves, but also by treatment of seeds with the disease controller, or absorption of the disease controller through the roots by its application to soil.

The applying dosage of the plant disease controller of the present invention is varied depending upon various factors such as a purpose, diseases to be controlled, a growth state of a plant, tendency of disease occurrence, weather, environmental conditions, a preparation form, an application method, an application site and application time. It may be properly chosen in the range of 0.1 g to 10 kg, in terms of the active ingredient, per 10 ares depending upon purposes.

The plant disease controller of the present invention may be used in admixture with other agricultural and horticultural disease controllers in order to expand both spectrum of controllable diseases and the period of time when effective applications are possible or to reduce the dosage.

### EXAMPLES

Example 1 is described below as an example of process for producing the compound of the present invention.

### Example 1

### Production of N-(3-chloro-4-methylphenyl)-4-methyl-1,2,3-thiadiazole-5-carboxamide

In 150 ml of tetrahydrofuran was dissolved 17 g (0.12 mol) of 3-chloro-4-methylaniline, followed by adding dropwise thereto 20 g (0.12 mol) of 4-methyl-1,2,3-thiadiazole-5-carboxylic acid chloride under ice-cooling over a period of 30 minutes. Then, 19 g (0.19 mol) of triethylamine was added dropwise thereto under ice-cooling over a period of 30 minutes. After completion of the dropwise addition, the reaction was carried out with stirring at room temperature for 2 hours. After completion of the reaction, a saturated aqueous sodium chloride solution was added to the reaction solution containing the desired compound, and the desired compound was extracted with ethyl acetate. The organic layer was washed successively with diluted hydrochloric acid, a saturated aqueous sodium hydrogencarbonate solution and a saturated aqueous sodium chloride solution, dried over anhydrous sodium sulfate, filtered and then concentrated under reduced pressure, and 33 g of the crude crystals thus obtained were recrystallized from hexane-isopropanol to obtain 29 g of the desired compound.

Physical property: m.p. 114°C.

Typical formulation examples and test examples of the present invention are described below but they should not be construed as limiting the scope of the invention.

In the examples, parts are all by weight.

| Formulation Example 1 | |
|---|---|
| The compound of the invention | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

| Formulation Example 2 | |
|---|---|
| The compound of the invention | 3 parts |
| Clay powder | 82 parts |
| Diatomaceous earth powder | 15 parts |

A dust was prepared by mixing uniformly and grinding the above ingredients.

| Formulation Example 3 | |
|---|---|
| The compound of the invention | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

| Formulation Example 4 | |
|---|---|
| The compound of the invention | 20 parts |
| Mixture of kaolin and synthetic, high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

### Test Example 1

### Controlling effect on rice blast by submerged application

A wettable powder containing the compound of the present invention as an active ingredient and wettable powders containing each reference compound as an active ingredient were prepared. Rice plants (cultivar: Kinnanpu) at the 6 leaf stage cultivated in a 1/10000-are pot were subjected to submerged application of each of the wettable powders in a predetermined dosage per 10 ares in terms of the active ingredient. After standing in a greenhouse for 1 week, the plants were inoculated with a suspension of spores of blast fungus (Pyricularia oryzae) by spraying. After the inoculation, the plants were allowed to stand at 20°C and a high humidity for 7 days to cause the disease sufficiently. Then, lesions in each leaf were counted and then compared with those on the untreated plot, and the control efficacy was calculated. The number of lesions on the untreated plot was 19 per leaf.

As the reference compounds, the following compounds described in JP-A-8-325110 were used.
A : N-phenyl-4-methyl-1,2,3-thiadiazole-5-carboxamide,
B : N-(3-chlorophenyl)-4-methyl-1,2,3-thiadiazole-5-carboxamide,
C : N-(4-methylphenyl)-4-methyl-1,2,3-thiadiazole-5-carboxamide,
D : N-(3,4-dichlorophenyl)-4-methyl-1,2,3-thiadiazole-5-carboxamide,
E : N-(3,4-dimethylphenyl)-4-methyl-1,2,3-thiadiazole-5-carboxamide.

The results obtained are shown in Table 1.

**Table 1**

| | Test compound | Dosage (g.ai/10a) | Control efficacy (%) |
|---|---|---|---|
| Invention plot | Compound of the present invention | 10 | 98 |
| | | 1 | 90 |
| Reference plot | A | 10 | 77 |
| | | 1 | 0 |
| | B | 10 | 80 |
| | | 1 | 30 |
| | C | 10 | 90 |
| | | 1 | 75 |
| | D | 10 | 85 |
| | | 1 | 57 |
| | E | 10 | 70 |
| | | 1 | 50 |

As shown in Table 1, the compound of the present invention was very effective in controlling rice blast at both dosages of 10 g and 1 g, in terms of the active ingredient, per 10 ares. On the other hand, the reference compounds A to E are rated A at a dosage of 200 g/10 a in terms of the active ingredient in the description given in JP-A-8-325110, page 49, the left column, line 24 to the right column, line 34. But as is shown in Table 1, their effect was lessened with a decrease of their dosage from 10 g to 1 g, and at a dosage of 1 g, the reference compounds A to E were remarkably inferior in the effect to the compound of the present invention.

### Test Example 2

### Controlling effect on rice blast and phytotoxicity test in a field

Rice plants (cultivar: Kinuhikari) raised in nursery beds were treated with a predetermined amount of granules prepared according to the formulation example, on the day of transplantation. The amount of the granules applied was 50 g per nursery bed. After the treatment, the rice plants were lightly sprinkled with water and transplanted in a regular rice paddy with a conventional transplanter.

The transplantation was carried out early in May and the effect and phytotoxicity were investigated 50 days after the treatment with the granules. The control efficacy was decided in the same manner as in Test Example 1. The phytotoxicity was decided by comparing the number of stems per plant with that on the untreated plot and was shown as a percentage of the former based on the latter. On the untreated plot, the number of stems was 21.4 per plant and the number of lesions 41.8 per plant.

The results obtained are shown in Table 2.

**Table 2**

| | Test compound | Number of stems (%) | Control efficacy (%) |
|---|---|---|---|
| Invention plot | Compound of the present invention | 101 | 98 |
| Reference plot | B | 72 | 74 |
| | C | 82 | 80 |
| | D | 90 | 72 |

As shown in Table 2, the compound of the present invention had no phytotoxicity and exhibited an excellent controlling effect. On the other hand, the reference compounds B, C and D were clearly inferior to the compound of the present invention in the controlling effect and had phytotoxicities such as the decrease of the number of stems. Thus, they finally exert a serious influence on the yield.

### Test Example 3

### Controlling effect on cucumber downy mildew fungus by spraying

A wettable powder containing the compound of the present invention as an active ingredient and wettable powders containing each reference compound as an active ingredient were diluted with water. Each of the dilutions was sprayed on cucumber plants (cultivar: Yotsuba) at the 1.5 leaf stage cultivated in a pot with a diameter of 9 cm, in a volume of 150 liters per 10 ares. The cucumber plants were cultivated in a green-house for 8 days and then inoculated with zoospores of cucumber downy mildew fungus (Pseudoperonospora cubensis) by spraying (the cucumber plants were at the 3.0 leaf stage at the time of the inoculation). After the inoculation, the cucumber plants were allowed to stand at 20°C and a high humidity for 7 days to cause the disease sufficiently. Then, lesions in the first to third leaves of each plant were counted and then compared with those on the untreated plot, and the control efficacy was calculated. The number of lesions on the untreated plot was 4 per leaf.

The results obtained are shown in Table 3.

**Table 3**

| | Test compound | Dosage (ppm) | Control efficacy (%) |
|---|---|---|---|
| Invention plot | Compound of the present invention | 200 | 96 |
| | | 20 | 65 |
| Reference plot | A | 200 | 46 |
| | | 20 | 0 |
| | C | 200 | 50 |
| | | 20 | 0 |

As shown in Table 3, the compound of the present invention retained a marked controlling effect at a concentration of 200 ppm even when the fungus was inoculated 8 days after the treatment with the compound.

### Test Example 4

### Controlling effect on wheat powdery mildew in a field

A wettable powder containing the compound of the present invention as an active ingredient and a wettable powder containing a reference compound as an active ingredient were diluted with water. Each of the dilutions was sprayed once on wheat (cultivar: Chihoku) sown late in September, in a volume of 100 liters per 10 ares earyly in May. In reference agent plots, Azoxystrobin (common name) was sprayed once in the middle of May and sulfur was sprayed twice in the middle of May and early in June. The area percentage of lesions in each cotyledon was measured and then compared with that on the untreated plot, and the control efficacy was calculated. The area percentage of lesions on the untreated plot was 17% per leaf.

The results are shown in Table 4.

**Table 4**

| | Test compound | Dosage (ppm) | Control efficacy (%) |
|---|---|---|---|
| Invention plot | Compound of the present invention | 400 | 100 |
| Reference plot | C | 400 | 24 |
| Reference agent plot | Azoxistrobin | 100 | 52 |
| | Sulfur | 1300 | 50 |

As shown in Table 4, when applied once in a concentration of 400 ppm, the compound of the present invention exhibited an excellent controlling effect as compared with the agents used for controlling wheat powdery mildew (Azoxystrobin applied once and sulfur applied twice) and the reference compound C.

## Claims

1. N-(3-chloro-4-methylphenyl)-4-methyl-1,2,3-thiadiazole-5-carboxamide.

2. A plant disease controller **characterized by** containing N-(3-chloro-4-methylphenyl)-4-methyl-1,2,3-thiadiazole-5-carboxamide as an active ingredient.

3. A method for controlling plant diseases, **characterized by** treating a crop to be protected, with an effective amount of a plant disease controller **characterized by** containing N-(3-chloro-4-methylphenyl)-4-methyl-1,2,3-thiadiazole-5-carboxamide as an active ingredient.

## Patentansprüche

1. N-(3-Chlor-4-methylphenyl)-4-methyl-1,2,3-thiadiazol-5-carboxamid.

2. Mittel zur Bekämpfung von Pflanzenkrankheiten, **dadurch gekennzeichnet, dass** es N-(3-Chlor-4-methylphenyl)-4-methyl-1,2,3-thiadiazol-5-carboxamid als aktiven Bestandteil enthält.

3. Verfahren zur Bekämpfung von Pflanzenkrankheiten, **gekennzeichnet durch** Behandeln einer zu schützenden Feldfrucht mit einer wirksamen Menge eines Mittels zur Bekämpfung von Pflanzenkrankheiten, **dadurch gekennzeichnet,** dass es N-(3-Chlor-4-methylphenyl)-4-methyl-1,2,3-thiadiazol-5-carboxamid als aktiven Bestandteil enthält.

## Revendications

1. N-(3-chloro-4-méthylphényl)-4-méthyl-1,2,3-thiadiazole-5-carboxamide.

2. Agent phytosanitaire **caractérisé en ce qu'**il contient du N-(3-chloro-4-méthylphényl)-4-méthyl-1,2,3-thiadiazole-5-carboxamide en tant qu'un ingrédient actif.

3. Méthode pour contrôler des maladies végétales, **caractérisée par** le traitement d'une culture à protéger, avec une quantité efficace d'un agent phytosanitaire **caractérisé en ce qu'**il contient du N-(3-chloro-4-méthylphényl)-4-méthyl-1,2,3-thiadiazole-5-carboxamide en tant qu'un ingrédient actif.
